# EUROPÄISCHE PATENTSCHRIFT

(11) **EP 0 682 941 B1**
(45) Veröffentlichungstag und Bekanntmachung des Hinweises auf die Patenterteilung: **07.01.1999**
(21) Anmeldenummer: 95104023.7
(22) Anmeldetag: 18.03.1995
(51) Int. Cl.: A61K 9/06

(54) **Salbengrundlage auf Kohlenwasserstoffbasis enthaltend Polydecenen**
Ointment carrier comprising hydrocarbons comprising polydecen
Véhicule pour onguent à base d'hydrocarbures comprenante polydecen

(30) Priorität: 19.05.1994 DE 4417640
(43) Veröffentlichungstag der Anmeldung: 22.11.1995
(73) Patentinhaber: TUDAPETROL MINERALÖLERZEUGNISSE NILS HANSEN KG, D-22297 Hamburg (DE)
(72) Erfinder: Hansen, Nils, D-25451 Quickborn (DE)
(74) Vertreter: Siewers, Gescha, Dr.

(56) Entgegenhaltungen:
- US-A- 2 628 187
- US-A- 4 948 580

## Beschreibung

Die Erfindung betrifft Salbengrundlagen auf Kohlenwasserstoffbasis mit Vaselinestruktur.

In der Pharmazie sind, zum Teil schon seit langem, Salbengrundlagen auf Kohlenwasserstoffbasis bekannt, deren am häufigsten verwendete wahrscheinlich Vaseline ist. Vaseline ist ein Produkt der Erdöldestillation und kommt in unterschiedlicher Qualität in den Handel. Vaseline besteht aus einem Gemisch im wesentlichen gesättigter und im wesentlichen geradkettiger Kohlenwasserstoffe bis hin zum Dotriacontan zusammen mit geringen Anteilen olefinischer Kohlenwasserstoffe mit etwa 10 bis 18 C-Atomen. Je nach Herkunft der Vaseline ist der Gehalt an Isoparaffinen verschieden hoch und die vorherrschenden n-Paraffine sind wesentlich fein kristalliner als die Iso-bzw. verzweigkettigen Paraffine. Zyklische und insbesondere aromatische Anteile sind allerdings in der Vaseline enthalten und können durch Entfärben nur teilweise entfernt werden.

Neben der Vaseline aus der Erdöldestillation sind auch sogenannte Kunstvaselinen bekannt, die aus einer Lösung von Ceresin oder Hartparaffinen in Paraffinöl bestehen und sich meistens von der aus Erdöl gewonnenen Vaseline durch Mängel in der Viskosität, Fadigkeit und Transparenz unterscheiden.

Als hydrophobe Zubereitung auf Kohlenwasserstoffbasis ist das sogenannte hydrophobe Basisgel bekannt, das aus medizinischem Praffinöl und Polyethylen besteht. Ein ähnliches Produkt ist die sogenannte Plastibase, die sich ebenfalls aus 95 Teilen flüssigen Paraffinen und 5 Teilen Polyethylen mit einem Molekulargewicht von etwa 20.000 zusammensetzt und deren Verwendung beispielsweise in der US-4,948,580 für schleimhautwirksame Zubereitungen beschrieben ist. Ähnliche Produkte sind beispielsweise in der US-2,628,187 offenbart, wobei dort in Mineralölen wie beispielsweise Polybutenen Polyethylene als Verdickungsmittel gelöst werden.

Gegen die Verwendung von Vaselinen oder Kohlenwasserstoffgel sind in letzter Zeit zunehmend Stimmen laut geworden, da sowohl Paraffine als auch Vaseline aus der Erdölfraktionierung stammen und naturgemäß gewisse Anteile an aromatischen und zwar auch polyaromatischen Verbindungen aufweisen. Es ist technisch mit zumutbarem Aufwand praktisch nicht möglich, diese Spuren von Polyaromaten aus Paraffinen oder Vaseline zu entfernen. Abgesehen davon, daß Vaseline und Paraffinsorten je nach Herkunft recht verschieden sind und daher auch unterschiedliche Mengen Aromaten enthalten, werden immer wieder Hautreizungen und andere Unverträglichkeitserscheinungen beobachtet und darüber hinaus besteht die Gefahr, daß die Zuführung von polyzyklischen Aromaten auf die Haut neoplastisches Wachstum auslösen könnte, und zwar insbesondere dann, wenn mit einer erhöhten UV-Strahlung und damit einer Erhöhung der Hautkrebsrate zu rechnen ist.

Der Erfindung liegt daher die Aufgabe zugrunde, eine Salbengrundlage auf Kohlenwasserstoffbasis zu entwickeln, die keine oder nur noch reduzierte Anteile an Bestandteilen enthält, die aus der Erdölfraktionierung gewonnen werden.

Zur Lösung der Aufgabe wird eine Salbengrundlage auf Kohlenwasserstoffbasis vorgeschlagen, die gekennzeichnet ist durch eine Lösung von Polyethylen hoher Dichte (PE-HD) oder niederer Dichte (PE-LD), Vinylestern oder -ethern oder kristallinen Paraffinen in Polydecenen.

Die vorgeschlagenen Polydecene sind Poly-Alphaolefine , die ein relativ niedriges Molekulargewicht im Molmassenbereich von etwa 300 - 3000 aufweisen und physiologisch unbedenklich sind. Polydecene werden, wie andere Poly-Alphaolefine, durch Polymerisation der gasförmigen Ausgangsprodukte wie Isodecen unter ionischen Bedingungen bei tiefer Temperatur hergestellt.

Zur Verdickung bis zur jeweils gewünschten Konsistenz werden den erfindungsgemäßen Salbengrundlagen entweder Polyethylen hoher Dichte PE-HD, also ein stark verzweigtes Polyethylen, PE-LD, also geringer Dichte oder Vinylester oder -ether zugesetzt oder gegebenenfalls kristalline n-Paraffine, die entweder bei der Erdölfraktionierung abgetrennt und vorgereinigt werden können oder die bei Kohleveredlungsverfahren anfallen. Vorzugsweise werden mikrokristalline Paraffine eingesetzt.

Da der Gehalt an kristallinen Paraffinen weniger als die Hälfte der Gesamtmasse ausmacht, kann auf diese Weise eine beträchtliche Reduzierung des Gehalts an aromatischen Verbindungen erfolgen oder die Salbengrundlagen sind sogar, wie bei der Verwendung von Polyethylen bis zur Nachweisgrenze aromatenfrei, da die synthetisch hergestellten Vinylpolymeren keine Aromaten enthalten.

Hinsichtlich der Viskosität, Fädigkeit und Transparenz entsprechen die erfindungsgemäßen Salbengrundlagen den Anforderungen der Arzneibücher für

Vaseline oder hydrophoben Baisgelen und sind daher aus dermatologischer Sicht ein vollwertiges Äquivalent für die bisher verwendeten Salbengrundlagen. Die Erfindung wird im Folgenden anhand der Beispiele näher erläutert:

### Beispiel 1

40 Gewichtsprozent kristalline Praffine mit einem Schmelzpunkt von 60° C werden in 60 Gewichtsprozent Polydecen unter Erwärmung gelöst. Das nach dem Erkalten erhaltene Produkt entspricht den Daten für Vaseline in den Arzneibüchern und ist in Viskosität, Fädigkeit und Transparenz voll äquivalent.

### Beispiel 2

5 Gewichtsprozent PE-LD mit einer Molmasse von etwa 80.000 und 5 Gewichtsprozent PE-HD mit einer Molmasse von etwas 60.000 werden in 90 Gewichtsprozent Polydecen unter Erwärmung gelöst.

### Beispiel 3

5 Gewichtsprozent PE-LD mit einer Molmasse von 100.00 werden in 95 Gewichtsprozent Polyisobuten mit einer Molmasse von etwa 2000 gelöst. Das entstandene Gel entspricht in dermatologischer Hinsicht dem üblichen hydrophoben Basisgel.

## Patentansprüche

1. Salbengrundlage auf Kohlenwasserstoffbasis, gekennzeichnet durch eine Lösung von Polyethylen hoher Dichte (PE-HD) oder niederer Dichte (PE-LD), Vinylestern oder -ethern oder kristallinen Paraffinen in Polydecenen.

2. Salbengrundlage nach Anspruch 1 dadurch gekennzeichnet, daß Polyethylene mit Molmassen bis 100.000 enthalten sind.

3. Salbengrundlage nach Anspruch 1 oder 2, dadurch gekennzeichnet, daß Polyvinylester oder -ether enthalten sind.

4. Salbengrundlage nach Anspruch 1 bis 3, dadurch gekennzeichnet, daß die Polydecene Molmassen im Bereich von etwa 300-3000 aufweisen.

5. Salbengrundlage nach Anspruch 1 bis 4, dadurch gekennzeichnet, daß das Gewichtsverhältnis von PE oder Vinylverbindungen oder kristallinen Paraffinen zu Polydecen etwa 10 zu 90 bis 40 zu 60 beträgt.

## Claims

1. Ointment base containing hydrocarbons characterized in a solution of polyethylenes of high (PE-HD) or low density (PE-LD), vinylesters or ethers or crystalline paraffins in polydecenes.

2. Ointment base according to claim 1 characterized in that it contains polyethylenes with molar masses up to 100.000.

3. Ointment base according to claim 1 or 2 characterized in that it contains polyvinylesters or ethers.

4. Ointment base according to claims 1 to 3 characterized in that the polydecenes have molar masses of about 300-3000.

5. Ointment base according to claims 1 to 4 characterized in that the weight relation of PE or vinyl compounds or crystalline paraffins and polydecenes is about 10 : 90 to 40 : 60.

## Revendications

1. Base d'onguent fondée sur des hydrocarbures caractérisée par une solution de polyéthylène de densité haute (PE-HD) ou basse (PE-LD), esters ou éthers de vinyle ou paraffines cristallines dans des polydécènes.

2. Base d'onguent selon revendication 1 caractérisée par une teneur en polyéthylène avec masses molaires jusqu'à 100.000.

3. Base d'onguent selon les revendications 1 ou 2 caractérisée par une teneur en éthers ou ester polyvinyliques.

4. Base d'onguent selon les revendications 1 jusqu'à 3 caractérisée par des polydécènes avec des masses molaires dans le domaine de 300-3000 environ.

5. Base d'onguent selon les revendications 1 jusqu'à 4 caractérisée par une relation des porids de PE, des composés vinyliques ou des paraffines cristallines et des polydédènes entre environ 10 jusqu'à 90 et 40 jusqu'à 60.
